# FASCICULE DE BREVET EUROPEEN

(11) **EP 1 185 314 B1**
(45) Date de publication et mention de la délivrance du brevet: **27.11.2002**
(21) Numéro de dépôt: 00951591.7
(22) Date de dépôt: 15.06.2000
(51) Int. Cl.: A61L 27/36, A61L 27/28, A61L 27/12

(54) **MATERIAU DE GREFFE OSSEUSE FORME D'UNE CERAMIQUE PHOSPHOCALCIQUE ET DE TISSU OSSEUX**
MATERIAL FÜR KNOCHENTRANSPLANTATE AUS CALCIUMPHOSPHAT-KERAMIK UND KNOCHENGEWEBE
BONE GRAFT MATERIAL FORMED OF CALCIUM PHOSPHATE CERAMICS AND BONE TISSUE

(30) Priorité: 15.06.1999 FR 9907571
(43) Date de publication de la demande: 13.03.2002
(73) Titulaire: Depuy Bioland, 31100 Toulouse (FR)
(72) Inventeur: Frayssinet, Patrick, 31470 Saiguede (FR); Mathon, Didier, 31470 Fonsorbes (FR)
(74) Mandataire: Bernasconi, Jean Raymond
(86) Numéro de dépôt international: FR0001661
(87) Numéro de publication internationale: WO00076560

(56) Documents cités:
- WO-A-97/14783
- KURASHINA K ET AL: "Experimental cranioplasty and skeletal augmentation using an alpha-tricalcium phosphate/dicalcium phosphate dibasic/tetracalcium phosphate monoxide cement: a preliminary short-term experiment in rabbits" BIOMATERIALS,GB,ELSEVIER SCIENCE PUBLISHERS BV., BARKING, vol. 19, no. 7-9, 5 avril 1998 (1998-04-05), pages 701-706, XP004120837 ISSN: 0142-9612
- KURASHINA K ET AL: "Osteogenesis in muscle with composite graft of hydroxyapatite and autogenous calvarial periosteum: a preliminary report" BIOMATERIALS,GB,ELSEVIER SCIENCE PUBLISHERS BV., BARKING, vol. 16, no. 2, 1 janvier 1995 (1995-01-01), pages 119-123, XP004033092 ISSN: 0142-9612

## Description

La présente invention concerne un matériau destiné à une greffe osseuse, formé d'une céramique phosphocalcique ayant été préalablement colonisée *in vivo* par des cellules de tissu osseux.

Le comblement de perte de substance osseuse est un problème quotidien en chirurgie orthopédique et traumatique ainsi qu'en cancérologie. Les matériaux de remplacement utilisés jusqu'à maintenant ont tous montré des limites à leur acceptation par le tissu osseux.

L'autogreffe de tissu osseux, quoique bien acceptée pour des volumes réduits, présente un intérêt limité du fait du peu de tissu disponible pour le prélèvement. Le prélèvement qu'elle impose entraîne une morbidité importante en raison de la douleur et du risque infectieux qu'elle induit.

L'allogreffe de tissu osseux de banque présente une recolonisation souvent réduite (Enneking, W.F., Journal of Bone and Joint Surgery 1991, 73-A, 8, 1123-1141) et un risque infectieux, notamment par le virus de l'hépatite B, le virus de l'immunodéficience acquise et le cytomégalovirus (Buck, B.E., Clinical Orthopaedic and Related Research 1990, 290: 249-253, Pereira, BJG, N. Engl. J. Med. 1991; 325: 454-60). Le risque immunologique n'est en outre pas négligeable et est responsable des ostéolyses massives pouvant être observées dans un certain nombre de cas.

Les matériaux synthétiques non résorbables d'origine polymérique induisent une réponse de rejet de la part des tissus implantés (Linder, L, et al J. Bone Joint Surg. 1983 (Br) 65: 646-49). Les phosphates de calcium d'origine naturelle ou synthétique ainsi que les carbonates de calcium d'origine corallienne paraissent très bien tolérés par le tissu osseux receveur (Jarcho, M, Clinical Orthopaedic and Related Research, 1981, 157, 259-277) et il s'établit même, en ce qui concerne l'hydroxyapatite (HA), un lien chimique entre le tissu osseux et le matériau. Ils sont néanmoins incapables d'induire une ossification dans une zone de tissu conjonctif indifférencié s'ils ne sont pas colonisés par des cellules engagées dans une différenciation ostéogénique.

Les composites organo-minéraux, formés d'un composé organique à base de collagène et de phosphate de calcium, souffrent des mêmes limites et présentent en outre un risque d'infection par des agents infectieux non conventionnels potentiellement transmissibles par le collagène.

Enfin, une famille de protéines appartenant à celle du TGF-β ("Transforming Growth Factor"), les protéines morphogénétiques osseuses, ont la propriété d'induire la formation de tissu osseux par voie enchondrale lorsqu'elles sont introduites dans un tissu d'origine mésenchymateuse. Ces protéines peuvent être associées à des matériaux divers pour leur conférer des propriétés ostéoinductrices.

Le tissu osseux est composé d'une matrice extracellulaire osseuse protéique associée a une matrice minérale constituée d'une apatite carbonatée de type B ou AB chez l'homme adulte (Legros, R., Thèse Institut National Polytechnique de Toulouse. Toulouse 1984). La matrice protéique est constituée de collagène de type I et de différentes protéines osseuses spécifiques (Termine, J.D.. In Cell and Molecular Biology of Vertebrate Hard Tissues. Ciba Foundation Symposium 136. John Wiley & sons. Avon 1988. pp 178-191).

Plusieurs mécanismes de minéralisation de la matrice extracellulaire osseuse, sous le contrôle de protéines osseuses, sont actuellement discutés. Les deux mécanismes qui prévalent aujourd'hui et qui ne s'excluent pas nécessairement ont localisé les nucléations initiales de cristaux de phosphates de calcium dans des vésicules matricielles (Arsenault, A., et al., Calcif Tissue Int (1991) 48: 46-55) ou dans des espaces existant entre les fibrilles de la fibre de collagène (Glimcher, M.J., (1985), In: Rubin, R.P., Weiss, G., Putney, J.W., (Eds) Calcium in Biological Systems. Plénum, New York, pp: 607-616). Dans les deux cas, des protéines osseuses spécifiques sont responsables de la nucléation des cristaux de phosphates et du contrôle de leur croissance (Mann, S., Nature 1988, 332: 119-124). Certaines de ces protéines contrôlent l'extension de la minéralisation hors de la fibre de collagène et hors des vésicules. Cette structure composite est responsable des propriétés mécaniques de l'os. Les protéines de la matrice extracellulaire responsables de la mise en place de la structure du tissu osseux sont synthétisées par des cellules spécialisées, les ostéoblastes. Ces derniers reçoivent et intègrent les informations émises sous forme de peptides ou protéines par les divers organes et cellules qui les constituent, pour adapter leur synthèse protéique aux besoins du tissu osseux qui dépendent de ces' informations (Frayssinet, P., Thèse Institut National Polytechnique de Toulouse. Toulouse 1990 - Francillon-Vieillot, H., et al, Skeletal Biomineralization: Patterns, Processes and Evolutionnary Trends. Vol I, J.G. Carter (ed), Van Nostrand Reinhold. New York 1990: 471-531). Ces cellules proviennent de précurseurs mésenchymateux présents dans le tissu stromal osseux se différenciant en plusieurs stades en ostéoblastes puis ostéocytes sous l'influence de divers facteurs protéiques dont certains font partie de la matrice extracellulaire osseuse à certains stades de son développement (Caplan, A.I., Journal of Ortopaedic Research 1991, 9: 641-650). La synthèse et le maintien de tissu osseux sont donc liés à la présence et à la fonctionnalité des cellules ostéogéniques et de leurs cellules souches.

Les cellules souches mésenchymateuses sont capables, après leur isolement et leur culture, de synthétiser un tissu osseux lorsqu'elles sont placées dans des chambres hémiperméabtes intrapéritonéales (Ashton, B.A., et al, Clin. Orthop. Rel. Res. 1980, 151: 294-307). Il a paru avantageux à divers auteurs d'utiliser les potentialités de synthèse et de différenciation des cellules ostéogéniques présentes dans le tissu osseux et son stroma afin d'induire une synthèse de tissu osseux dans des zones où le besoin existe et où la faillite des possibilités de synthèses de l'individu a été constatée (Friedenstein, A.J., et al, Exp. Hematol. 1982, 10, 2: 217-227). Celle-ci est habituellement due à une absence locale de cellules ostéogéniques différenciées. Il faut noter que l'implantation de matériaux de substitution de tissu osseux ne pallie pas le manque de cellules ostéogéniques. La supplémentation en cellules à différenciation ostéogénique a été faite sous forme d'injection de suspension de cellules médullaires non sélectionnées (Conolly, J., et al, J. Bone Joint Surg. 1989, 71A, 5: 684-691) ou bien sous forme de cellules medullaires également non sélectionnées et adsorbées de manière extemporanée, lors de l'acte chirurgical, sur un support phosphocalcique (Ohgushi, H., et al, Journal of Orthopaedics Research 1983, 7: 568-578).

Il est possible de cultiver des cellules ostéogéniques hors de l'organisme en leur gardant un état de différenciation tel qu'elles soient capables de synthétiser, lors de la culture, une matrice extracellulaire qui se minéralise *in vitro* (Gerstenfeld, L.C., Developmental Biology 1987, 122, 49-60).

Il est également possible de cultiver ces cellules *in vitro* sur des supports qui seront ensuite implantés dans un site nécessitant un apport de substance osseuse.

Néanmoins, la culture de cellules différenciées présente des inconvénients. Les cellules perdent leurs caractères différenciés et acquièrent des anomalies chromosomiques. En outre, l'isolement de cellules spécifiquement ostéogéniques est aléatoire. Il résulte de ceci que la formation de tissu osseux *in vitro* ou *in vivo* à partir de prélèvement de cellules ostéogéniques est lui-même aléatoire. D'autre part, la matrice osseuse pouvant être formée par des cellules isolées *in vitro* est de structure différente de la structure du tissu osseux obtenu *in vivo.* Le tissu osseux formé *in vitro* ne présente pas de structure lamellaire, de bordure cellulaire ni de structures vasculaires.

Ollier, dans son "Traité expérimental et clinique de la régénération des os et de la production artificielle du tissu osseux" (Victor Masson et Fils, Paris, 1868), a montré chez divers animaux que le périoste dévascularisé pouvait être réimplanté ectopiquement et former du tissu osseux. L'enroulement de céramiques phosphocalciques dans des bouts de périostes vascularisés a également permis de produire du tissu osseux (Kurashina et al., 1995 ; Ishida et al., 1995).

Afin d'éliminer les inconvénients liés au passage *in vitro* de cellules isolées, les auteurs de la présente invention ont mis au point un matériau destiné à une greffe osseuse, formé d'une céramique phosphocalcique ayant été préalablement colonisée *in vivo* par des cellules de tissu osseux, obtenu par le procédé comprenant les étapes consistant à :
- implanter au moins une céramique phosphocalcique poreuse *in vivo* sous le périoste d'un individu, au contact de la couche basale composée de cellules ostéogéniques ;
- laisser la céramique sous le périoste le temps que celle-ci soit colonisée par des cellules ostéogéniques ; et
- explanter la céramique ainsi colonisée.

La présente invention a pour objet le matériau biocompatible et fonctionnel obtenu par le procédé ci-dessus.

L'expression "préalablement colonisée" signifie que la colonisation de la céramique par les cellules du tissu osseux n'a pas lieu au site de la greffe mais dans un autre site du corps humain ou animal.

Le matériau ainsi obtenu peut alors être réimplanté dans un site nécessitant l'apport d'une greffe osseuse.

L'invention permet d'obtenir un matériau prêt à être réimplanté, qui ne contient pas de périoste mais des:cellules qui sont engagées dans une voie de différenciation ostéogénique. Le tissu formé dans les pores de la céramique contient des cellules et du collagène, et de la substance ostéoïde.

Ce tissu est minéralisé et on peut observer la présence de capillaires, cette vascularisation étant capable de s'anastomoser à celle du site d'implantation.

La présente invention a également pour objet l'utilisation d'un matériau selon la revendication 1 pour la préparation d'un support de greffe osseuse destiné à être réimplanté dans un site osseux nécessitant une greffe.

De manière préférentielle, les cellules de tissu osseux qui colonisent la céramique phosphocalcique sont des cellules autologues.

La céramique phosphocalcique utilisée peut être n'importe quelle céramique poreuse connue de l'homme du métier pour ses propriétés de biocompatibilité.

Parmi les céramiques d'intérêt, on peut notamment citer celles à base d'hydroxyapatite (HA) et/ou de phosphate tricalcique β (β-TCP). Par exemple, une céramique contenant de 0 à 75 % d'HA et 25 à 100 % de β-TCP peut être utilisée. Une céramique comprenant environ 75 % d'HA et 25 % de β-TCP est préférée.

On préfère une céramique ayant une porosité de 40 à 90 %, de préférence 70 %. Une céramique avec des pores d'une taille entre 200 et 400 µm peut être par ailleurs avantageusement utilisée.

Il est en outre avantageux que les pores de la céramique soient interconnectés pour être tous accessibles à une invasion cellulaire.

Conformément à la présente invention, une céramique phosphocalcique est colonisée *in vivo* par des cellules de tissu osseux, telles que des cellules ostéogéniques, qui se fixent sur la céramique et se différencient sur celle-ci. A cette fin, la céramique peut être implantée sous le périoste au contact de la couche basale de n'importe quel os, de préférence dans un site facile d'accès, tel que par exemple l'épine de l'omoplate.

La lésion du périoste entraînée par l'implantation des céramiques déclenche la multiplication et la différenciation ostéogénique des cellules souches basales qui envahissent la porosité de la céramique.

La période pendant laquelle la céramique est maintenue implantée sous le périoste doit être suffisamment longue pour permettre l'envahissement de la céramique par des cellules ostéogéniques sans toutefois qu'une ossification extensive n'empêche le prélèvement.

Une période de 15 à 25 jours, de préférence de 18 à 20 jours, est pour cela préférée. Cette période d'implantation permet l'établissement d'un réseau vasculaire dans la porosité de la céramique. Le tissu ostéogénique présent dans la céramique peut survivre coupé de ses afférences vasculaires et implanté dans un autre site à l'issue de cette période de chargement et différenciation *in vivo*.

Les exemples et la figure suivante illustrent l'invention sans en limiter la portée.

### LEGENDE DES FIGURES :

La figure 1 annexée est un graphe représentant l'évolution de la porosité (% de la surface de section de l'implant occupée par les pores) et de l'ossification (% de la surface de section des pores occupées par de l'os) durant l'implantation musculaire de céramiques phosphocalciques préparées selon l'exemple 1.

La figure 2 annexée est un graphe représentant l'évolution de la résistance à la compression de céramiques prélevées à différents temps, selon l'exemple 2.

### EXEMPLES

### Exemple 1 : Implantation de céramiques poreuses de phosphate de calcium sous le périoste de l'omoplate de moutons adultes et réimplantation dans les muscles paravertébraux

### Matériels et méthodes :

Les céramiques à base de phosphate de calcium utilisées sont constituées de 75 % d'hydroxyapatite et de 25 % de β-TCP avec une porosité de 70 % et des tailles de pores entre 200 et 400 µm. Elles ont la forme d'un parallélépipède de 20x10x5 mm.

### Protocole d'implantation :

Ces céramiques sont implantées sous le périoste de brebis adultes le long de l'épine de l'omoplate. 4 céramiques sont placées de chaque côté pour 18 ou 25 jours. Elles sont ensuite prélevées et réimplantées individuellement dans les muscles paravertébraux de chaque coté de la colonne dorsale. Les céramiques sont prélevées à différents temps : 0, 15, 30 et 45 jours d'implantation.

### Protocole histologique :

Les implants sont ensuite analysés histologiquement, après fixation et déshydratation dans l'éthanol.

Les implants sont imprégnés dans de l'hydroxyéthylméthacrylate (HEMA). Deux de ces implants sont déminéralisés dans. une solution de Na-EDTA 10 % et imprégnés de HEMA. Des coupes de 5 µm sont obtenues et les coupes non déminéralisées sont colorées pour révéler leur activité phosphatase alcaline et par la méthode de Von Kossa. Les coupes sont contre-colorées au giemsa.

### Histomorphométrie :

La porosité de la céramique et le rapport surfacique des pores occupés par le tissu osseux ont été évalués par analyse d'image.

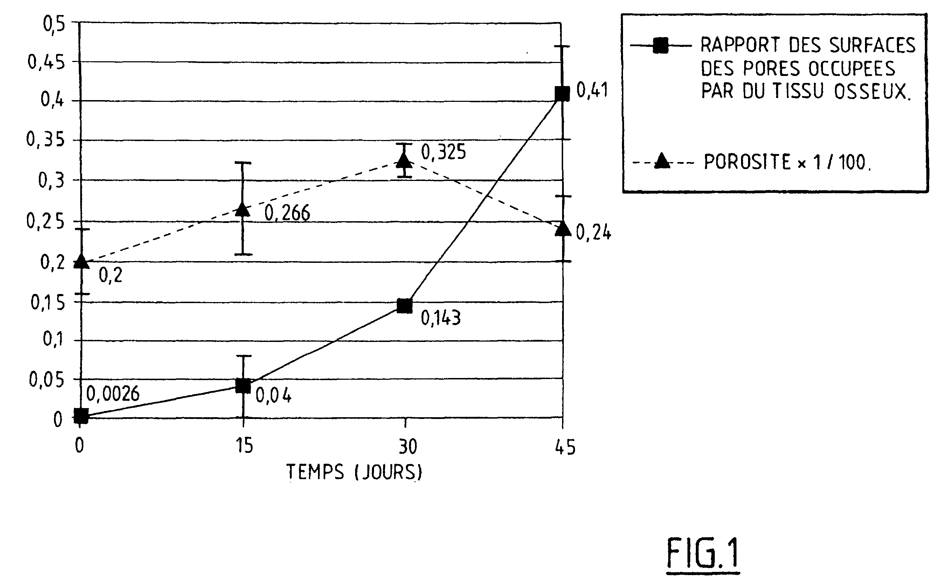

Il ressort que, après prélèvement au temps 0, (18 ou 25 jours après l'implantation sous le périoste) une très faible formation de trabécules osseux peut-être mise en évidence à la surface de la céramique en contact avec le tissu osseux. La très grosse majorité du volume des pores est occupée par du tissu conjonctif lâche. Quinze jours après l'implantation dans les muscles, quelques trabécules osseux sont visibles dans le volume des pores. La porosité ne varie pas pendant toute la durée d'implantation de 0 à 45 jours et la quantité de tissu osseux augmente régulièrement pendant toute la durée d'implantation alors que l'arrivée de cellules ostéogéniques n'est pas possible (figure 1). Cela signifie qu'il existe une charge de cellules ostéogéniques ayant pénétré dans le matériau lors de l'implantation sous le périoste et que ces cellules vont se différencier durant toute la durée de l'implantation.

### Exemple 2 : Etude mécanique

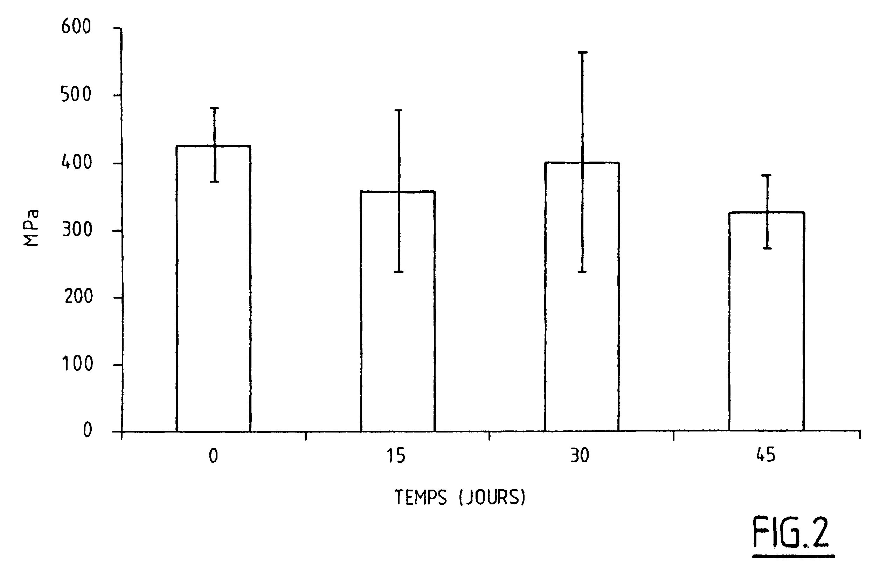

Comme dans l'exemple 1, des céramiques rectangulaires (5 x 10 x 20 mm) sont implantées sous le périoste de brebis adultes le long de l'épine de l'omoplate. 4 céramiques sont placées de chaque côté pour 18 ou 25 jours. Elles sont ensuite prélevées et réimplantées individuellement dans les muscles paravertébraux de chaque coté de la colonne dorsale. Les céramiques sont prélevées à différents temps : 0, 15, 30 et 45 jours d'implantation. Leur résistance à la compression est testée (figure 2) et les fragments obtenus sont ensuite analysés histologiquement et la surface des pores ainsi que celle occupée par le tissu osseux sont déterminées. Il est apparu que, comme dans l'expérience précédente, le volume des pores occupé par de l'os progresse tout au long de l'implantation. La résistance mécanique des céramiques n'est pas corrélée à la quantité de tissu osseux présent dans les pores. La résistance mécanique n'a pas varié significativement durant le temps d'implantation dans les muscles.

## Revendications

1. Matériau destiné à une greffe osseuse, formé d'une céramique phosphocalcique ayant été préalablement colonisée *in vivo* par des cellules de tissu osseux, obtenu par le procédé comprenant les étapes consistant à :
- implanter au moins une céramique phosphocalcique poreuse *in vivo* sous le périoste d'un individu, au contact de la couche basale composée de cellules ostéogéniques ;
- laisser la céramique sous le périoste le temps que celle-ci soit colonisée par des cellules ostéogéniques ; et
- explanter la céramique ainsi colonisée.

2. Matériau selon la revendication 1 dans lequel la céramique phosphocalcique a été préalablement colonisée par des cellules de tissu osseux par implantation *in vivo* sous le périoste au contact de la couche basale, pendant une période de 15 à 25 jours puis explantation.

3. Matériau selon l'une quelconque des revendications 1 ou 2 formé d'une céramique à base d'hydroxyapatite (HA) et/ou de phosphate tricalcique β (β-TCP).

4. Matériau selon l'une quelconque des revendications précédentes, formé d'une céramique phosphocalcique ayant une porosité de 40 à 90 %, de préférence 70 %.

5. Matériau selon l'une quelconque des revendications précédentes, formé d'une céramique phosphocalcique dont les pores ont une taille entre 200 et 400 µm.

6. Utilisation d'un matériau selon la revendication 1 destiné à être réimplanté dans un site osseux nécessitant une greffe.

7. Utilisation selon la revendication 6, dans laquelle le matériau est formé d'une céramique à base d'hydroxyapatite (HA) et/ou de phosphate tricalcique β (β-TCP).

8. Utilisation selon l'une quelconque des revendications 6 ou 7, dans laquelle le matériau est formé d'une céramique phosphocalcique ayant une porosité de 40 à 90 %, de préférence 70 %.

9. Utilisation selon l'une quelconque des revendications 6 à 8, dans laquelle le matériau est formé d'une céramique phosphocalcique dont les pores ont une taille entre 200 et 400 µm.

## Patentansprüche

1. Material für Knochentransplate aus zunächst in vivo durch Knochengewebszellen besiedelter Kalziumphospatkeramik, welches durch das folgende Schritte aufweisende Verfahren erhalten worden ist:
- Implantation in vivo mindestens einer porösen Kalziumphosphatkeramik unter das ,im Kontakt mit der aus osteogenen Zellen bestehenden Basalschicht befindliche Periost einer Person;
- Belassen der Keramik solange unter dem Periost bis diese durch die osteogenen Zellen besiedelt worden ist ; und
- Explantation der so besiedelten Keramik.

2. Material nach Anspruch 1, bei dem die Kalziumphosphatkeramik zunächst durch Implantion in vivo unter dem Periost im Kontakt mit der Basalschicht befindliche Knochengewebszellen während einer Zeitdauer von 15 bis 25 Tagen besiedelt worden ist .

3. Material nach irgendeinem der Ansprüche 1 oder 2, welches aus einer Keramik auf der Basis von Hydroxylapatit (HA) und / oder β-Trikalziumphosphat (β - TCP) gebildet worden ist.

4. Material nach irgendeinem der vorangehenden Ansprüche, welches aus einer Kalziumphosphatkeramik mit einer Porosität von 40 bis 90 %, bevorzugt 70 % gebildet worden ist.

5. Material nach irgendeinem der vorangehenden Ansprüche , welches aus einer Kalziumphosphatkeramik gebildet worden ist, dessen Poren Abmessungen zwischen 200 und 400 µm aufweisen.

6. Benutzung eines Materials nach Anspruch 1, welches dazu bestimmt ist, an einem eine Transplantation erfordernden aus Knochen bestehenden Ort reimplaniert zu werden.

7. Benutzung eines Materials nach Ansprch 6, bei dem das Material aus einer Keramik auf der Basis von Hydroxylapatit (HA) und / oder β-Trikalziumphosphat (β - TCP) gebildet worden ist.

8. Benutzung nach irgendeiner der Ansprüche 6 oder 7, bei der das Material aus einer Kalziumphosphatkeramik mit einer Porosität von 40 bis 90 %, bevorzugt 70 % gebildet worden ist

9. Benutzung nach irgendeiner der Ansprüche 6 bis 8, bei der das Material aus einer Kalziumphosphatkeramik gebildet ist, dessen Poren Abmessungen zwischen 200 und 400 µm aufweisen.

## Claims

1. Material intended for a bone graft, formed from a calcium phosphate ceramic which has been previously colonized in vivo by bone tissue cells, obtained by the process comprising the stages consisting of:
- implanting at least one porous calcium phosphate ceramic in vivo beneath the periosteum of an individual, in contact with the basal layer formed from osteogenic cells,
- leaving the ceramic beneath the periosteum for the time during which it is colonized by osteogenic cells and
- explanting the thus colonized ceramic.

2. Material according to claim 1, wherein the calcium phosphate ceramic has been previously colonized by bone tissue cells by in vivo implantation beneath the periosteum in contact with the basal layer, for a period of 15 to 25 days, followed by explantation.

3. Material according to either of the claims 1 and 2, formed from a ceramic based on hydroxyapatite (HA) and/or β-tricalcium phosphate (β-TCP).

4. Material according to any one of the preceding claims, formed from a calcium phosphate ceramic having a porosity of 40 to 90%, preferably 70%.

5. Material according to any one of the preceding claims, formed from a calcium phosphate ceramic, whose pores have a size between 200 and 400 µm.

6. Use of a material according to claim 1 intended to be reimplanted in a bone site requiring a graft.

7. Use according to claim 6, wherein the material is formed by a ceramic based on hydroxyapatite (HA) and/or β-tricalcium phosphate (β-TCP).

8. Use according to either of the claims 6 and 7, wherein the material is formed by a calcium phosphate ceramic having a porosity of 40 to 90%, preferably 70%.

9. Use according to any one of the claims 6 to 8, wherein the material is formed by a calcium phosphate ceramic, whose pores have a size between 200 and 400 µm.
